# EUROPEAN PATENT SPECIFICATION

(11) **EP 2 199 856 B1**
(45) Date of publication and mention of the grant of the patent: **07.08.2013**
(21) Application number: 08172167.2
(22) Date of filing: 18.12.2008
(51) Int. Cl.: G03F 7/038, C07C 381/12, C07D 303/28, C07D 305/06, C07D 335/16, G03F 7/004

(54) **Cationic radiation curable compositions**
Kationische strahlungshärtbare Zusammensetzungen
Compositions durcissables par radiation cationique

(43) Date of publication of application: 23.06.2010
(73) Proprietor: Agfa Graphics N.V., 2640 Mortsel (BE)
(72) Inventor: Loccufier, Johan, 2640, Mortsel (BE)

(56) References cited:
- EP-A- 0 510 839
- WO-A-2008/040648
- JP-A- 8 041 116
- US-B1- 6 395 450
- MAX PLANCK: "The theory of heat radiation" 1988, SPRINGER , XP002530044 ISBN: 0883185970 * page 16 *

## Description

### Technical Field

The present invention relates to a cationic radiation curable composition, especially suitable for printing or coating on food packaging and toys, comprising at least one polymerizable sulfonium initiator.

### Background Art

It is generally stated in radiation curable technology that cationic radiation curable compositions are more suited for printing or coating on food packaging, compared to free radical radiation curable composition, due to the complete conversion of the monomers. This should lead to very low amounts of residual monomer compared to free radical compositions. However, the photoinitiators, such as triaryl sulfoniums and diaryl iodoniums, used in cationic radiation curable compositions, remain migratable as such, if they are not fully converted by exposure to radiation, as well as their degradation products, which are often toxic, such as benzene, and malodourous such as diphenylsulfide. To solve this issue, several approaches have been disclosed in he prior art.

One approach to minimize the extraction of photoinitiators is the use of photoinitiators with a higher molecular weight. However, polymeric initiators have a tendency to lose reactivity. Multifunctional and polymeric sulfonium salts have been disclosed in GB 2396153 (SUN CHEMICAL) and in WO 03/072568 (SUN CHEMICAL). Upon fragmentation by exposure, both proposed sulfoniums still yield migratable fragments, such as e.g. isopropyl thioxanthone.

In another approach, specific substitution patterns on sulfonium initiators have been proposed, to avoid the formation of toxic compounds such as benzene and reduce the formation of volatile compounds, upon fragmentation by exposure, to avoid odour. Fused ring sulfoniums have been disclosed in WO 03/072567 (SUN CHEMICAL). Ester substituted sulfoniums have been disclosed in WO 2007/003507 (CIBA). Specific hexaaryl sulfoniums have been disclosed in WO 2007/118794 (CIBA). Though optimized for volatility, the decomposition products remain migratable.

EP 0510839 A (CIBA) discloses triaryl sulfoxonium salts being substituted on one of the aryl rings by a substituent comprising a polymerizable functional group to reduce migration. Though being a solution for unconverted residual initiators, several decomposition products still remain migratable. Difunctional sulfoxonium initiators, substituted by four epoxides, have been disclosed in JP 08041116 (NIPPON KAYAKU). These initiators are improved for low odour but still yield migratable decomposition products.

US 6395450 (Samsung) discloses a positive working photoresist composition including at least one thermally cross-linkable photoacid generator, a binder polymer, and a solvent in which the binder polymer and photoacid generator are dissolved.

Therefore, there is still a need for sulfonium initiators, further optimized for reduced migration of both residual initiator and the corresponding decomposition products.

### Disclosure of Invention

### Objects of the Invention

It is an object of the present invention to provide a cationic radiation curable composition, especially suitable for printing on food packaging and toys, comprising at least one polymerizable sulfonium initiator and one polymerizable monomer, oligomer or prepolymer.

It is a further object of the present invention to provide a cationic radiation curable ink composition, especially suitable for printing on food packaging and toys, comprising at least one polymerizable sulfonium initiator and one polymerizable monomer, oligomer or prepolymer.

It is a further object of the present invention to provide a cationic radiation curable ink jet ink composition, especially suitable for printing on food packaging and toys, comprising at least one polymerizable sulfonium initiator and one polymerizable monomer, oligomer or prepolymer.

These and other objects of the present invention will become apparent from the description hereinafter.

### Summary of the Invention

It has been found that a cationic radiation curable composition comprising at least one sulfonium initiator according to Formula (I) or (II), and one or more monomers, oligomers and prepolymers selected from the group consisting of epoxides, oxetanes and vinyl ethers, further substantially free of organic solvent gives no or a very low amount of migratable residues of unreacted initiator nor of its degradation products and is especially suitable for printing or coating on food packaging and toys.

Objects of the invention have been realized with a radiation curable composition including a polymerizable photoinitiator as defined by claim 1.

Further advantages and embodiments of the present invention will become apparent from the following description.

### Definitions

The term "dye", as used in disclosing the present invention, means a colorant having a solubility of 10 mg/L or more in the medium in which it is applied and under the ambient conditions pertaining.

The term "pigment" is defined in DIN 55943, as a colorant that is practically insoluble in the application medium under the pertaining ambient conditions, hence having a solubility of less than 10 mg/L therein.

The term "C.I." is used in disclosing the present application as an abbreviation for Color Index.

The term "cationic photoinitiator" is used in disclosing the present application as having the same meaning as a photo-acid generator.

The term "alkyl" means all variants possible for each number of carbon atoms in the alkyl group i.e. for three carbon atoms: n-propyl and isopropyl; for four carbon atoms: n-butyl, isobutyl and tertiary-butyl; for five carbon atoms: n-pentyl, 1,1-dimethyl-propyl, 2,2-dimethylpropyl and 2-methyl-butyl etc.

### Radiation Curable Compositions

The radiation curable composition according to the present invention contains a cationic photoinitiator according to Formula (I) or (II): 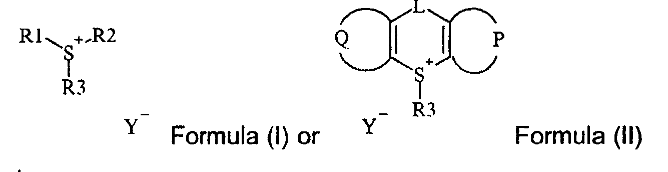 wherein :
R1, R2 and R3 independently represent a group selected from the group consisting of an aryl group and a heteroaryl group, with the proviso that R1, R2 and R3 are each substituted by at least one substituent comprising at least one cationically polymerizable group selected from the group consisting of an alkenyl ether, an epoxide and an oxetane;
P and Q independently represent the necessary atoms to form a substituted or unsubstituted 5 or 6-membered aromatic or heteroaromatic ring, with the proviso that at least one of P,Q and L is substituted by at least one substituent comprising at least one cationically polymerizable group, selected from the group consisting of an alkenyl ether, an epoxide and an oxetane;
L represents a group selected from the group consisting of a direct bond, O, S, SO, SO₂, CR5R6, C(=O), S(=O), C(NR8) and NR7;
R5 and R6 independently represent a group selected from the group consisting of a hydrogen, a substituted or unsubstituted alkyl group, a substituted or unsubstituted alkenyl group a substituted or unsubstituted alkynyl group, a substituted or unsubstituted aralkyl group, a substituted or unsubstituted alkaryl group, a substituted or unsubstituted aryl group, a substituted or unsubstituted heteroaryl group, a halogen, an alcohol, an ether, an ester, an amine, an amide, a carboxylic acid, a thiol and thioether;
R5 and R6 may represent the necessary atoms to form a five or six membered ring;
R7 and R8 independently represent a group selected from the group consisting of a hydrogen, an substituted or unsubstituted alkyl group, a substituted or unsubstituted alkenyl group a substituted or unsubstituted alkynyl group, a substituted or unsubstituted aralkyl group, a substituted or unsubstituted alkaryl group, a substituted or unsubstituted aryl group, a substituted or unsubstituted heteroaryl group and an acyl group; and
Y- represents a counter ion to compensate for the positive charge of the sulfonium compound.

Y- represents an anion. In general, there is no particular limitation on the nature of the anion to be used. However, the anion should be non-nucleophilic, or essentially non-nucleophilic, as is well known in the art. It should also be relatively bulky.

Examples of non-nucleophilic anions are well known to those skilled in the art, and include anions of formula MZₙ- where M represents a phosphorus, boron, antimony, arsenic, chlorine or carbon atom, Z represents a halogen atom except where M represents a halogen atom, an oxygen atom or a sulphite group, and n is an integer dependent upon the valence of M and Z. Preferred examples of such groups include the PF₆-, SbF₆-, BF₄-, B(C₆F₅)₄-, R^{a}B(Ph)₃ (where R^{a} represents an alkyl group having from 1 to 6 carbon atoms and Ph represents a phenyl group), R^{b}S0₃ (where R^{b} represents an alkyl or haloalkyl group having from 1 to 6 carbon atoms or an aryl group), Cl0₄- and ArSO₃- (where Ar represents an aryl group) groups, of which the PF₆-, SbF₆-, AsF₆-, CF₃SO₃- and BF₄- groups are preferred and the PF₆- group is most preferred.

Preferred examples for Y- as an organic or inorganic anion are non-nucleophilic anions, selected from the group (BZ₄)-, (SbZ₆)-, (PZ₆)-, (B(C₆Z₅)₄)-, with Z denoting a halogen, in particular F or Cl, preferably F; C₁-C₂₀-alkylsulphonate, C₁-C₂₀-haloalkylsulphonate, C₁-C₂₀-perfluoroalkylsulphonate, unsubstituted C₆-C₁₀-arylsulphonate, camphorsulphonate, C₁-C₂₀-perfluoroalkylsulphonylmethide, C₁-C₂₀-perfluoroalkylsulphonylimide, and C₆-C₁₀-arylsulphonate substituted by halogen, NO₂, SO₃M, C₁-C₁₂-alkyl, C₁-C₁₂-haloalkyl, C₁-C₁₂-alkoxy, phenylsulphonyloxy, C₁-C₄-alkylphenylsulphonyloxy or by COOR_{z}; wherein R_{z} is C₁-C₂₀-alkyl, phenyl, benzyl; or phenyl mono- or polysubstituted by C₁-C12-alkyl, C₁-C₁₂-alkoxy or by halogen and M is as defined above.

C₁-C₂₀-Alkylsulphonate is RₓSO₃- wherein Rₓ is linear or branched C₁-C₂₀-alkyl as described above. Examples thereof include methylsulphonate, ethylsulphonate, propylsulphonate, pentylsulphonate and hexylsulphonate.

C₂-C₂₀-Haloalkylsulphonate is RₓSO₃- wherein Rₓ is halo-substituted C₂-C₂₀-alkyl, C₂-C₁₀-, C₂-C₈- or C₄-C₈-alkyl. Examples thereof include C₂F₅SO₃-, C₄F₉SO₃-and C₈F₁₇SO₃-. C₆-C₁₀-Arylsulphonate is RₓSO₃ wherein Rₓ is C₆-C₁₀-aryl, e.g. phenyl or naphthyl. Alkyl-substituted arylsulphonates are, for example, toluenesulphonate, 2,4,6-trimethylbenzene-sulphonate, 2,4,6-tris(isopropyl)benzenesulphonate, 4-tert-butylbenzenesulphonate and 4-dodecylbenzenesulphonate. Halo-substituted arylsulphonates are, for example, 4-chlorobenzenesulphonate, 4-fluoro- benzenesulphonate, 2,4,6-trifluorobenzenesulphonate and pentafluorobenzenesulphonate.

In a preferred embodiment, R1, R2 and R3 all represent a phenyl group each substituted by at least one substituent comprising at least one cationically polymerizable group selected from the group consisting of an alkenyl ether, an epoxide and an oxetane.

In a preferred embodiment, the 6-membered aromatic rings formed through the atoms P and Q both represent a phenyl group.

A radiation curable composition, comprising a sulfonium initiator according to Formula (I) is particularly preferred.

In a further preferred embodiment, the cationically polymerizable group is selected from the group consisting of an epoxide group and a vinyl ether group, a vinyl ether group being particularly preferred.

Typical structures according to the present invention are given below in Table 1 without being limited thereto.

**Table 1**

| | |
|---|---|
| 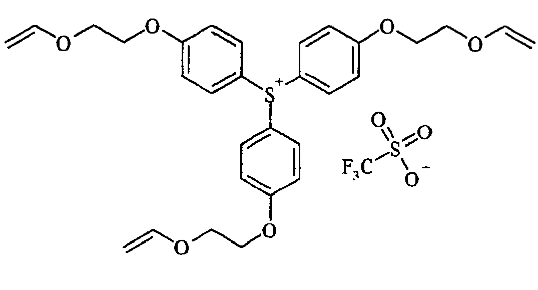 | Sulfonium-1 |
| 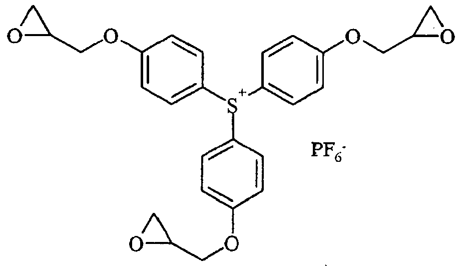 | Sulfonium-2 |
| 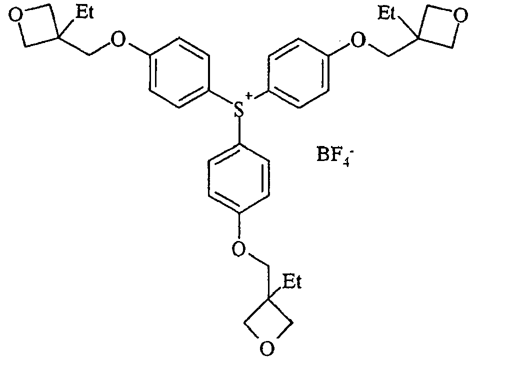 | Sulfonium-3 |
| 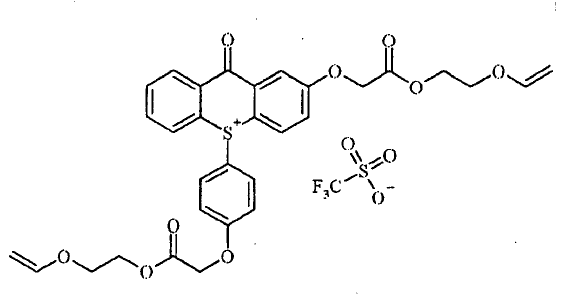 | Sulfonium-4 |
| 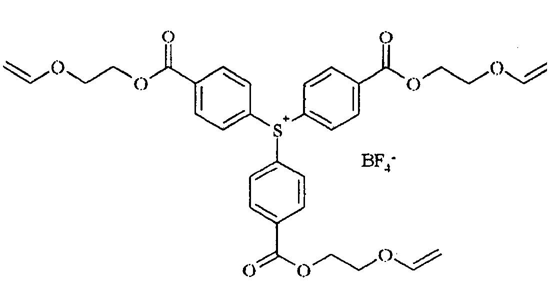 | Sulfonium-5 |
| 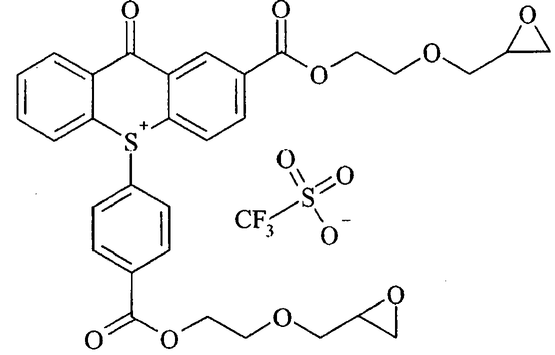 | Sulfonium-6 |
| 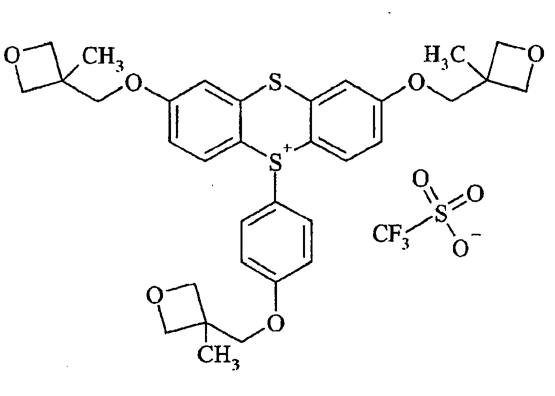 | Sulfonium-7 |
| 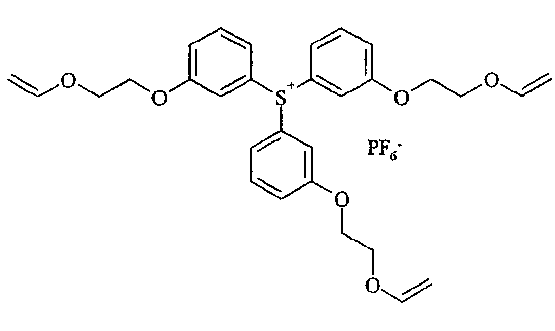 | Sulfonium-8 |
| 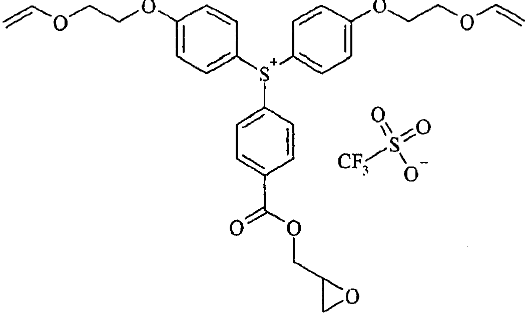 | Sulfonium-9 |
| 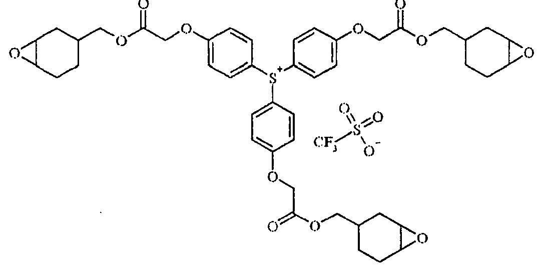 | Sulfonium-10 |

The radiation curable composition can be used as a colourless liquid or as a coloured liquid, in the latter case it is called a radiation curable ink.

In a preferred embodiment, the radiation curable composition according to the present invention is a radiation curable inkjet composition, more preferably a radiation curable inkjet ink.

The radiation curable compositions and inks can also be advantageously used in offset printing, screen printing, flexographic printing and other printing or coating techniques.

The radiation curable composition according to the present invention is substantially free of organic solvent.

The radiation curable compositions and inks do not contain an evaporable component such as an organic solvent.

The radiation curable compositions and inks are preferably part of an ink set, more preferably an inkjet ink set, comprising at least one ink containing one or more colorants, preferably one or more colour pigments. The curable ink set preferably comprises at least one yellow curable ink (Y), at least one cyan curable ink (C) and at least one magenta curable ink (M) and preferably also at least one black curable ink (K). The curable CMYK-ink set may also be extended with extra inks such as red, green, blue, and/or orange to further enlarge the colour gamut of the image. The CMYK-ink set may also be extended by the combination of full density and light density inks of both colour inks and/or black inks to improve the image quality by lowered graininess.

The pigmented radiation curable ink preferably contains a dispersant, more preferably a polymeric dispersant, for dispersing the pigment. The pigmented curable ink may contain a dispersion synergist to improve the dispersion quality and stability of the ink. Preferably, at least the magenta ink contains a dispersion synergist. A mixture of dispersion synergists may be used to further improve dispersion stability.

The viscosity of the curable liquid and ink is preferably smaller than 100 mPa.s at 30°C and at a shear rate of 100 s⁻¹. The viscosity of the radiation curable inkjet inks and liquids is preferably smaller than 50 mPa.s, more preferably lower than 30 mPa.s, and most preferably between 2 and 15 mPa.s at a shear rate of 100 s⁻¹ and a jetting temperature between 10 and 70°C.

The surface tension of the curable liquid and ink is preferably in the range of about 20 mN/m to about 70 mN/m at 25°C, more preferably in the range of about 22 mN/m to about 40 mN/m at 25°C.

The curable composition or ink may further also contain at least one stabilizer for improving the thermal stability of composition or ink.

The curable composition or ink may further also contain at least one surfactant for obtaining good spreading characteristics on a substrate.

### Sensitizers

It is known in the art that photoinitiated cationic polymerizations are wavelength tuneable, by combining classical photoinitiators such as sulfonium salts with specific photosensitizers, absorbing light in the desired spectral range. Typical photosensitizers are selected from the group consisting of anthracene derivatives, as disclosed by Crivello et al. (Macromol. Symp., 217, 47-61 (2004)), thioxanthone derivatives as disclosed by Takahashi et al. (Journal of Photopolymer Science and Technology, 12(1), 147-152 (1999) and Cho et al. (European Polymer Journal, 41, 367-374 (2005)), phenothiazine derivatives as disclosed by Kunze et al. (Journal of Photochemistry and Photobiology, A: Chemistry, 110(2), 115-122 (1997)) and perylene derivatives as disclosed by Aydogan et al. in Photochemistry and UV Curing (2006), 187-201 (Editor: J.P. Fouassier).

In the present invention, any spectral sensitizer, known in the art can be used. Thioxanthone derivatives are particularly preferred photosensitizers in the present invention.

### Monomers, Oligomers and Prepolymers

The one or more monomers, oligomers and prepolymers used in the radiation curable composition of the invention are selected from the group consisting of epoxides, oxetanes and vinyl ethers.

It is possible, for example, to use all customary epoxides, such as aromatic, aliphatic or cycloaliphatic epoxy resins. These are compounds having at least one, preferably at least two, epoxy group(s) in the molecule. Examples thereof are the glycidyl ethers and [beta]-methyl glycidyl ethers of aliphatic or cycloaliphatic diols or polyols, e.g. those of ethylene glycol, propane-1 ,2-diol, propane-1 ,3-diol, butane-1 ,4-diol, diethylene glycol, polyethylene glycol, polypropylene glycol, glycerol, trimethylolpropane or 1 ,4-dimethylolcyclohexane or of 2,2-bis(4-hydroxycyclohexyl)propane and N,N-bis(2-hydroxyethyl)aniline; the glycidyl ethers of di- and poly-phenols, for example of resorcinol, of 4,4'-dihydroxyphenyl-2,2-propane, of novolaks or of 1 ,1 ,2,2-tetrakis(4-hydroxyphenyl)ethane. Examples thereof include phenyl glycidyl ether, p-tert-butyl phenyl glycidyl ether, o-cresyl glycidyl ether, polytetrahydrofuran glycidyl ether, n-butyl glycidyl ether, 2-ethylhexylglycidylether, C₁₂-isoalkyl glycidyl ether and cyclohexanedimethanol diglycidyl ether. Further examples include N-glycidyl compounds, for example the glycidyl compounds of ethylene urea, 1,3-propyleneurea or 5-dimethyl-hydantoin or of 4,4'-methylene- 5,5'-tetramethyldihydantoin, or compounds such as triglycidyl isocyanurate.

Further examples of glycidyl ether components that can be used in the radiation curable compositions according to the invention are, for example, glycidyl ethers of polyhydric phenols obtained by the reaction of polyhydric phenols with an excess of chlorohydrin, such as, for example, epichlorohydrin (e.g. glycidyl ethers of 2,2-bis(2,3-epoxypropoxyphenol)propane. Further examples of glycidyl ether epoxides that can be used in connection with the present invention are described, for example, in US 3018262 (SHELL OIL) and in "Handbook of Epoxy Resins" by Lee and Neville, McGraw-Hill Book Co., New York (1967).

There is also a large number of commercially available glycidyl ether epoxides that are suitable, such as, for example, glycidyl methacrylate, diglycidyl ethers of bisphenol A, for example those obtainable under the trade names EPON™ 828, EPON™ 825, EPON™ 1004 and EPON™ 1010 (Shell); DER-331 , DER-332 and DER-334 (Dow Chemical); 1 ,4-butanediol diglycidyl ethers of phenolformaldehyde novolak, e.g. DEN-431 , DEN-438 (Dow Chemical); and resorcinol diglycidyl ethers; alkyl glycidyl ethers, such as, for example, C₈-C₁₀glycidyl ethers, e.g. HELOXY™ Modifier 7, C₁₂-C₁₄glycidyl ethers, e.g. HELOXY™ Modifier 8, butyl glycidyl ethers, e.g. HELOXY™ Modifier 61 , cresyl glycidyl ethers, e.g. HELOXY™ Modifier 62, p-tert-butylphenyl glycidyl ethers, e.g. HELOXY Modifier 65, polyfunctional glycidyl ethers, such as diglycidyl ethers of 1 ,4-butanediol, e.g. HELOXY™ Modifier 67, diglycidyl ethers of neopentyl glycol, e.g. HELOXY™ Modifier 68, diglycidyl ethers of cyclohexanedimethanol, e.g. HELOXY™ Modifier 107, trimethylolethane triglycidyl ethers, e.g. HELOXY™ Modifier 44, trimethylolpropane triglycidyl ethers, e.g. HELOXY™ Modifier 48, polyglycidyl ethers of aliphatic polyols, e.g. HELOXY™ Modifier 84 (all HELOXY™ glycidyl ethers are obtainable from Shell). Also suitable are glycidyl ethers that comprise copolymers of acrylic esters, such as, for ex- ample, styrene-glycidyl methacrylate or methyl methacrylate-glycidyl acrylate. Examples thereof include 1:1 styrene/glycidyl methacrylate, 1:1 methyl methacrylate/glycidyl acrylate, 62.5:24:13.5 methyl methacrylate/ethyl acrylate/glycidyl methacrylate. The polymers of the glycidyl ether compounds can, for example, also comprise other functionalities provided that these do not impair the cationic curing. Other suitable glycidyl ether compounds that are commercially available are polyfunctional liquid and solid novolak glycidyl ether resins, e.g. PY™ 307, EPN™ 1179, EPN™ 1180, EPN™ 1182 and ECN™ 9699.

Suitable vinyl ethers include aromatic, aliphatic or cycloaliphatic vinyl ethers and also silicon-containing vinyl ethers. These are compounds having at least one, preferably at least two, vinyl ether groups in the molecule. Examples of vinyl ethers suitable for use in the compositions according to the invention include triethylene glycol divinyl ether, 1 ,4-cyclohexanedimethanol divinyl ether, 4-hydroxybutyl vinyl ether, the propenyl ether of propylene carbonate, dodecyl vinyl ether, tert-butyl vinyl ether, tert-amyl vinyl ether, cyclohexyl vinyl ether, 2-ethylhexyl vinyl ether, ethylene glycol monovinyl ether, butanediol monovinyl ether, hexanediol monovinyl ether, 1 ,4-cyclohexanedimethanol monovinyl ether, diethylene glycol monovinyl ether, ethylene glycol divinyl ether, ethylene glycol butyl vinyl ether, butane-1 ,4-diol divinyl ether, hexanediol divinyl ether, diethylene glycol divinyl ether, triethylene glycol divinyl ether, triethylene glycol methyl vinyl ether, tetra-ethylene glycol divinyl ether, pluriol-E-200 divinyl ether, polytetrahydrofuran divinyl ether-290, trimethylolpropane trivinyl ether, dipropylene glycol divinyl ether, octadecyl vinyl ether, (4-cyclohexyl- methyleneoxyethene)-glutaric acid methyl ester and (4-butoxyethene)-isophthalic acid ester.

A preferred class of monomers and oligomers are vinyl ether acrylates such as those described in US 6310115 (AGFA). Particularly preferred compounds are 2- (2-vinyloxyethoxy)ethyl (meth)acrylate, most preferably the compound is 2- (2-vinyloxyethoxy)ethyl acrylate.

Preferred oxetane compounds suitable as monomer, oligomer and prepolymer in the radiation curable compositions and inks according to the present invention can be found in US 20050119362 (KONICA), especially the oxetane compounds listed in to . It is preferred to combine an epoxy compound and an oxetane compound to raise the reaction rate.

The monomers, oligomers and prepolymers are preferably present in the range of 60 to 95 % by weight, preferably in the range of 70 to 90 % by weight, each based on the total weight of radiation curable composition.

### Colorants

Colorants used in the radiation curable inks may be dyes, pigments or a combination thereof. Organic and/or inorganic pigments may be used. The colorant is preferably a pigment or a polymeric dye, most preferably a pigment.

The pigments may be black, white, cyan, magenta, yellow, red, orange, violet, blue, green, brown, mixtures thereof, and the like. This colour pigment may be chosen from those disclosed by HERBST, Willy, et al. Industrial Organic Pigments, Production, Properties, Applications. 3rd edition. Wiley - VCH , 2004. ISBN 3527305769.

Suitable pigments are disclosed in paragraphs to of WO 2008/074548 (AGFA GRAPHICS).

Suitable pigments include mixed crystals of the above particular preferred pigments. Mixed crystals are also referred to as solid solutions. For example, under certain conditions different quinacridones mix with each other to form solid solutions, which are quite different from both physical mixtures of the compounds and from the compounds themselves. In a solid solution, the molecules of the components enter into the same crystal lattice, usually, but not always, that of one of the components. The x-ray diffraction pattern of the resulting crystalline solid is characteristic of that solid and can be clearly differentiated from the pattern of a physical mixture of the same components in the same proportion. In such physical mixtures, the x-ray pattern of each of the components can be distinguished, and the disappearance of many of these lines is one of the criteria of the formation of solid solutions. A commercially available example is Cinquasia™ Magenta RT-355-D from Ciba Specialty Chemicals.

Also mixtures of pigments may be used in the radiation curable inks. For some inkjet applications, a neutral black inkjet ink is preferred and can be obtained, for example, by mixing a black pigment and a cyan pigment into the ink. The inkjet application may also require one or more spot colours, for example for packaging inkjet printing or textile inkjet printing. Silver and gold are often desired colours for inkjet poster printing and point-of-sales displays.

Non-organic pigments may be used in the colour inkjet inks. Particular preferred pigments are C.I. Pigment Metal 1, 2 and 3. Illustrative examples of the inorganic pigments include red iron oxide (III), cadmium red, ultramarine blue, prussian blue, chromium oxide green, cobalt green, amber, titanium black and synthetic iron black.

Pigment particles in inkjet inks should be sufficiently small to permit free flow of the ink through the inkjet-printing device, especially at the ejecting nozzles. It is also desirable to use small particles for maximum colour strength and to slow down sedimentation.

The numeric average pigment particle size is preferably between 0.050 and 1 µm, more preferably between 0.070 and 0.300 µm and particularly preferably between 0.080 and 0.200 µm. Most preferably, the numeric average pigment particle size is no larger than 0.150 µm. An average particle size smaller than 0.050 µm is less desirable for decreased lightfastness, but mainly also because very small pigment particles or individual pigment molecules thereof may still be extracted in food packaging applications. The average particle size of pigment particles is determined with a Brookhaven Instruments Particle Sizer BI90plus based upon the principle of dynamic light scattering. The ink is diluted with ethyl acetate to a pigment concentration of 0.002 wt%. The measurement settings of the BI90plus are: 5 runs at 23°C, angle of 90°, wavelength of 635 nm and graphics = correction function.

However for a white radiation curable ink, the numeric average particle diameter of the white pigment is preferably from 50 to 500 nm, more preferably from 150 to 400 nm, and most preferably from 200 to 350 nm. Sufficient hiding power cannot be obtained when the average diameter is less than 50 nm, and the storage ability and the jet-out suitability of the ink tend to be degraded when the average diameter exceeds 500 nm. The determination of the numeric average particle diameter is best performed by photon correlation spectroscopy at a wavelength of 633 nm with a 4mW HeNe laser on a diluted sample of the pigmented inkjet ink. A suitable particle size analyzer used was a Malvern™ nano-S available from Goffin-Meyvis. A sample can be, for example, be prepared by addition of one drop of ink to a cuvet containing 1.5 mL ethyl acetate and mixed until a homogenous sample was obtained. The measured particle size is the average value of 3 consecutive measurements consisting of 6 runs of 20 seconds.

Suitable white pigments are given by Table 2 in of WO 2008/074548 (AGFA GRAPHICS). The white pigment is preferably a pigment with a refractive index greater than 1.60. The white pigments may be employed singly or in combination. Preferably titanium dioxide is used as pigment with a refractive index greater than 1.60. Suitable titanium dioxide pigments are those disclosed in and in of WO 2008/074548 (AGFA GRAPHICS).

The pigments are present in the range of 0.01 to 10 % by weight, preferably in the range of 0.1 to 5 % by weight, each based on the total weight of radiation curable ink. For white radiation curable inks, the white pigment is preferably present in an amount of 3% to 30% by weight of the ink composition, and more preferably 5% to 25%. An amount of less than 3% by weight cannot achieve sufficient covering power and usually exhibits very poor storage stability and ejection property.

Generally pigments are stabilized in the dispersion medium by dispersing agents, such as polymeric dispersants. However, the surface of the pigments can be modified to obtain so-called "self-dispersible" or "self-dispersing" pigments, i.e. pigments that are dispersible in the dispersion medium without dispersants.

### Dispersants

The dispersant is preferably a polymeric dispersant. Typical polymeric dispersants are copolymers of two monomers but may contain three, four, five or even more monomers. The properties of polymeric dispersants depend on both the nature of the monomers and their distribution in the polymer. Suitable copolymeric dispersants have the following polymer compositions:
- statistically polymerized monomers (e.g. monomers A and B polymerized into ABBAABAB);
- alternating polymerized monomers (e.g. monomers A and B polymerized into ABABABAB);
- gradient (tapered) polymerized monomers (e.g. monomers A and B polymerized into AAABAABBABBB);
- block copolymers (e.g. monomers A and B polymerized into AAAAABBBBBB) wherein the block length of each of the blocks (2, 3, 4, 5 or even more) is important for the dispersion capability of the polymeric dispersant;
- graft copolymers (graft copolymers consist of a polymeric backbone with polymeric side chains attached to the backbone); and
- mixed forms of these polymers, e.g. blocky gradient copolymers.

Suitable dispersants includes the Solsperse™ series by NOVEON and the PB™ series by Ajinomoto Fine Techno Corp. Particularly preferred dispersants are Solsperse™ 32000, 35000 and 39000 dispersants from NOVEON.

The polymeric dispersant has preferably a number average molecular weight Mn between 500 and 30000, more preferably between 1500 and 10000.

The polymeric dispersant has preferably a weight average molecular weight Mw smaller than 100000, more preferably smaller than 50000 and most preferably smaller than 30000.

The polymeric dispersant has preferably a polydispersity PD smaller than 2, more preferably smaller than 1.75 and most preferably smaller than 1.5.

The polymeric dispersant is preferably used in an amount of 2 to 600 wt%, more preferably 5 to 200 wt% based on the weight of the pigment.

### Stabilizers

In this invention, a heat-induced base generator can be also utilized to improve ejection stability and storage stability.

As heat-induced base generator, for example, salt of organic acid, which decomposes by decarboxylation with heat, and base; a compound which release amines by decomposition via a reaction such as intramolecular nucleophilic substitution, Lossen rearrangement and Beckman's rearrangement, and those releasing base by causing some reaction with heat can be preferably utilized. Specifically, listed are salt of trichloroacetic acid described in GB 998949 (KODAK) , salt of α-sulfonylacetic acid described in US 4060420 (KODAK) , aldoxime carbamate derivatives and 2-carboxycarboxyamide derivatives described in EP 118078 A (FUJI) , salt utilizing alkali metal or alkaline earth metal other than organic base as a base component with thermal decomposing acid described in EP 120661 A (FUJI) , hydroxame carbamates utilizing Lossen rearrangement described in EP 123937 A (FUJI) , and aldoxime carbamates which generate nitrile with heat described in EP 123937 A (FUJI).

Further specific examples include guanidine trichloroacetate, methylguanidine trichloroacetate, potassium trichloroacetate, guanidine phenylsulfonylacetate, guanidine p-chlorophenylsulfonylacetate, guanidine p-methanesulfonylphenylsulfonylacetate, potassium phenylpropiolate, guanidine phenylpropiolate, cesium phenylpropiolate, guanidine p-chlorophenylpropiolate, guanidine p-phenylene-bisphenylpropiolate, tetramethylammonium phenylsulfonylacetate and tetramethylammonium phenylpropiolate.

The cationic polymerizable composition of the present invention may also contain 0.1 to 5% by weight of water based on the total weight of the radiation curable composition, in order to obtain sufficient storage stability.

### Surfactants

A surfactant may be appropriately incorporated to adjust the surface tension. A preferred surfactant is an anionic surfactant such as dialkylsulfosuccinates, alkylnaphthalenesulfonates and fatty acid salts; non-ionic surfactants such as polyoxyethylene alkyl ethers, polyoxyethylene alkylallyl ethers, acetylene glycols and polyoxyethylene-polyoxypropylene block copolymers; cationic surfactants such as alkylamines and quaternary ammonium salts; and surface active compounds having a polymerizing group. Among them, specifically preferred are surface active compounds having a polymerizing group such as an unsaturated bond and an oxirane or oxetane ring, such as silicone modified acrylate, fluorine modified acrylate, silicone modified epoxy, fluorine modified epoxy, silicone modified oxetane and fluorine modified oxetane.

### Preparation of Radiation Curable Inks

The average particle size and distribution is an important feature for inkjet inks. The ink may be prepared by precipitating or milling the pigment in the dispersion medium in the presence of the dispersant.

Mixing apparatuses may include a pressure kneader, an open kneader, a planetary mixer, a dissolver, and a Dalton Universal Mixer. Suitable milling and dispersion apparatuses are a ball mill, a pearl mill, a colloid mill, a high-speed disperser, double rollers, a bead mill, a paint conditioner, and triple rollers. The dispersions may also be prepared using ultrasonic energy.

Many different types of materials may be used as milling media, such as glasses, ceramics, metals, and plastics. In a preferred embodiment, the grinding media can comprise particles, preferably substantially spherical in shape, e.g. beads consisting essentially of a polymeric resin or yttrium stabilized zirconium oxide beads.

In the process of mixing, milling and dispersion, each process is performed with cooling to prevent build up of heat, and for radiation curable inks as much as possible under light conditions in which actinic radiation has been substantially excluded.

The ink may contain more than one pigment, the ink may be prepared using separate dispersions for each pigment, or alternatively several pigments may be mixed and co-milled in preparing the dispersion.

The dispersion process can be carried out in a continuous, batch or semi-batch mode.

The preferred amounts and ratios of the ingredients of the mill grind will vary widely depending upon the specific materials and the intended applications. The contents of the milling mixture comprise the mill grind and the milling media. The mill grind comprises pigment, polymeric dispersant and a liquid carrier. For inkjet inks, the pigment is usually present in the mill grind at 1 to 50 wt%, excluding the milling media. The weight ratio of pigment over polymeric dispersant is 20:1 to 1:2.

The milling time can vary widely and depends upon the pigment, selected mechanical means and residence conditions, the initial and desired final particle size, etc. In the present invention pigment dispersions with an average particle size of less than 100 nm may be prepared.

After milling is completed, the milling media is separated from the milled particulate product (in either a dry or liquid dispersion form) using conventional separation techniques, such as by filtration, sieving through a mesh screen, and the like. Often the sieve is built into the mill, e.g. for a bead mill. The milled pigment concentrate is preferably separated from the milling media by filtration.

In general it is desirable to make the inks in the form of a concentrated mill grind, which is subsequently diluted to the appropriate concentration for use in the printing system. This technique permits preparation of a greater quantity of pigmented ink from the equipment. By dilution, the ink is adjusted to the desired viscosity, surface tension, colour, hue, saturation density, and print area coverage for the particular application.

### Inkjet Printing Means

Curable compositions and inks according to the present invention may be jetted by one or more print heads ejecting small droplets of ink in a controlled manner through nozzles onto an ink-receiver surface, which is moving relative to the print head(s).

A preferred print head for the inkjet printing system is a piezoelectric head. Piezoelectric inkjet printing is based on the movement of a piezoelectric ceramic transducer when a voltage is applied thereto. The application of a voltage changes the shape of the piezoelectric ceramic transducer in the print head creating a void, which is then filled with ink. When the voltage is again removed, the ceramic expands to its original shape, ejecting a drop of ink from the print head. However the inkjet printing method according to the present invention is not restricted to piezoelectric inkjet printing. Other inkjet print heads can be used and include various types, such as a continuous type and thermal, electrostatic and acoustic drop on demand type.

At high printing speeds, the inks must be ejected readily from the print heads, which puts a number of constraints on the physical properties of the ink, e.g. a low viscosity at the jetting temperature, which may vary from 25°C to 110°C, a surface energy such that the print head nozzle can form the necessary small droplets, a homogenous ink capable of rapid conversion to a dry printed area,...

The inkjet print head normally scans back and forth in a transversal direction across the moving ink-receiver surface. Often the inkjet print head does not print on the way back. Bi-directional printing is preferred for obtaining a high areal throughput. Another preferred printing method is by a "single pass printing process", which can be performed by using page wide inkjet print heads or multiple staggered inkjet print heads which cover the entire width of the ink-receiver surface. In a single pass printing process the inkjet print heads usually remain stationary and the ink-receiver surface is transported under the inkjet print heads.

### Curing means

Curable compositions and inks according to the present invention can be cured by exposing them to actinic radiation, preferably by ultraviolet radiation.

In inkjet printing, the curing means may be arranged in combination with the print head of the inkjet printer, travelling therewith so that the curable composition is exposed to curing radiation very shortly after been jetted.

In such an arrangement it can be difficult to provide a small enough radiation source connected to and travelling with the print head. Therefore, a static fixed radiation source may be employed, e.g. a source of curing UV-light, connected to the radiation source by means of flexible radiation conductive means such as a fiber optic bundle or an internally reflective flexible tube.

Alternatively, the actinic radiation may be supplied from a fixed source to the radiation head by an arrangement of mirrors including a mirror upon the radiation head.

The source of radiation arranged not to move with the print head, may also be an elongated radiation source extending transversely across the ink-receiver surface to be cured and adjacent the transverse path of the print head so that the subsequent rows of images formed by the print head are passed, stepwise or continually, beneath that radiation source.

Any ultraviolet light source, as long as part of the emitted light can be absorbed by the photo-initiator or photo-initiator system, may be employed as a radiation source, such as, a high or low pressure mercury lamp, a cold cathode tube, a black light, an ultraviolet LED, an ultraviolet laser, and a flash light. Of these, the preferred source is one exhibiting a relatively long wavelength UV-contribution having a dominant wavelength of 300-400 nm. Specifically, a UV-A light source is preferred due to the reduced light scattering therewith resulting in more efficient interior curing.

UV radiation is generally classed as UV-A, UV-B, and UV-C as follows:
- UV-A: 400 nm to 320 nm
- UV-B: 320 nm to 290 nm
- UV-C: 290 nm to 100 nm.

Furthermore, it is possible to cure the image using, consecutively or simultaneously, two light sources of differing wavelength or illuminance. For example, the first UV-source can be selected to be rich in UV-C, in particular in the range of 260 nm-200 nm. The second UV-source can then be rich in UV-A, e.g. a gallium-doped lamp, or a different lamp high in both UV-A and UV-B. The use of two UV-sources has been found to have advantages e.g. a fast curing speed and a high curing degree.

The UV curing may be combined with heat curing. The advantage and of using heat is disclosed e.g. in Radiation Curing of Coatings, by Joseph V. Koleske, published by ASTM International, West Conshohocken, PA, USA,2002, ISBN 0-8031-2095-8, more specifically page 123 on the effect of adding thermal energy.

### EXAMPLES

### Materials

All materials used in the following examples were readily available from Aldrich Chemical Co. (Belgium) unless otherwise specified.

**VEEA** is 2-(2-vinyloxyethoxy)ethyl acrylate, a difunctional monomer available from Nippon Shokubai, Japan: 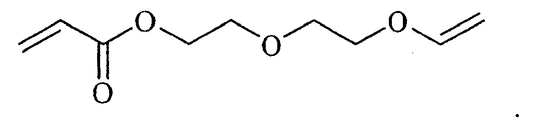 **Uvacure™ 1503** is a mixture of alkyl glycidyl ethers and (3,4-epoxycyclohexyl)methyl-3,4-epoxycyclohexyl carboxylate, supplied by Cytec Industries.
**ITX** is an abbreviation used for Genocure™ ITX, an isomeric mixture of 2-and 4-isopropylthioxanthone, a supplied by Rahn.
**Silwet™ L7602** is a silicon surfactant, supplied by Osi Specialties.
**PB15:4** is an abbreviation used for Hostaperm™ Blue P-BFS, a cyan pigment (C.I. Pigment Blue 15:4) available from CLARIANT.
**SP35000** is an abbreviation used for SOLSPERSE™ 35000, a solvent free polyethyleneimine-polyester hyperdispersant from NOVEON. **Genorad™16** is a polymerization inhibitor from RAHN AG.
**COMPINI-1** is tris[4-(2-ethoxyethoxy)phenyl]sulfonium triflate and was prepared as follows: 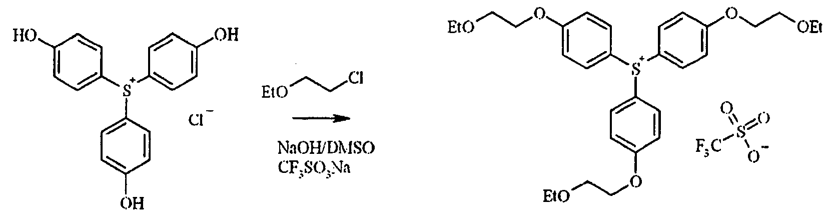 5.19 g (15.0 mmol) tris[4-hydroxyphenyl]sulfonium chloride was dissolved in 13 mL dimethyl sulfoxide. 3.58 g (89.5 mmol) sodium hydroxide was added and the mixture was stirred for 1 hour at 60°C under an argon atmosphere. 14.8 mL (14.65 g, 135 mmol) (2-chloroethyl)ethyl ether was added to the slurry and the mixture was heated to 85°C for 6 hours. The reaction mixture was allowed to cool down to room temperature and 160 mL diethyl ether and 160 mL water were added. The aqueous fraction was isolated and saturated with sodium chloride. The mixture was extracted twice with 80 mL nitromethane. The pooled nitromethane fractions were washed with 65 mL brine, dried over Na₂SO₄ and evaporated under reduced pressure. The residue was dissolved in 15 mL methanol and a solution of 6.2 g (36.0 mmol) sodium triflate in 29 mL methanol was added. The mixture was left standing over night. The precipitated sodium chloride was removed by filtration and the methanol solution was evaporated to half its volume. 85 mL water was added and the mixture was extracted twice with 60 mL ethyl acetate. The pooled organic fractions were dried over Na₂SO₄ and evaporated under reduced pressure. tris[4-(2-ethoxyethoxy)phenyl]sulfonium triflate was further purified on Kieselgel 60, using chloroform/methanol 4/1 as eluent. The isolated tris[4-(2-ethoxyethoxy)phenyl]sulfonium triflate was dissolved in 30 mL ethyl acetate and extracted with a solution of 1.5 g sodium triflate in 15 mL water. The organic fraction was dried over Na₂SO₄ and evaporated under reduced pressure. 2.00 g (24 %) of tris[4-(2-ethoxyethoxy)phenyl]sulfonium triflate was isolated as a viscous oil. **PET100 substrate** is a 100 µm subbed polyethylene terephthalate substrate with on the backside an antiblocking layer with antistatic properties available from AGFA-GEVAERT as P100C S/AS (Type 198).

### Methods of Measurement

### 1. Average particle size

The average particle size of pigment particles in inkjet ink was determined with a Brookhaven Instruments Particle Sizer BI90plus based upon the principle of dynamic light scattering. The ink or dispersion was diluted with ethyl acetate to a pigment concentration of 0.002 wt%. The measurement settings of the BI90plus were: 5 runs at 23°C, angle of 90°, wavelength of 635 nm and graphics = correction function.

For good ink jet characteristics (jetting characteristics and print quality) the average particle size of the dispersed particles should be less than 200 nm, preferably less than 150 nm.

### 2. Viscosity

The viscosity of the inkjet inks was measured using a Brookfield DV-II+ viscometer at 25°C and shear rate of 4 RPM using a CPE 40 spindle.

### 3. Curing degree

The curing degree is tested on a coating immediately after curing with UV light. The cured coating is rubbed with the means of a Q-tip. When the surface is not damaged, the coating is fully cured. When some of the cured coating can be damaged, the coating is only partly cured. When the whole cured coating is damaged, the coating is not cured.

### Example 1

This example illustrates the synthesis of photoinitiators for the radiation curable composition.

### Synthesis of tris[4-(2-vinyloxyethoxy)phenyl]sulfonium triflate (sulfonium-1)

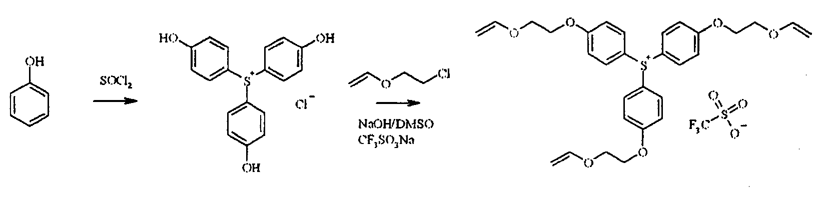

### Synthesis of tris[4-hydroxyphenyl]sulfonium chloride :

75 g (0.8 mol) phenol was dissolved in 40 mL chloroform. 15.3 mL (25 g, 0.21 mol) thionyl chloride was added drop wise, while the temperature was kept between 20 and 23°C. The reaction was allowed to continue for 48 hours at room temperature. The solvent and the excess of phenol were removed under reduced pressure (1 Torr at 90°C). The oily residue treated twice with 120 mL diethyl ether and four times with 50 mL acetone. Upon treating with acetone, tris[4-hydroxyphenyl]sulfonium chloride crystallized. Tris[4-hydroxyphenyl]sulfonium chloride was isolated by filtration and dried. 12.46 g (17%) tris[4-hydroxyphenyl]sulfonium chloride was isolated (m.p. 262-266°C).

### Synthesis of tris[4-(2-vinyloxyethoxy)phenyl]sulfonium triflate (Sulfonium-1)

4.93 g (14.3 mmol) tris[4-hydroxyphenyl]sulfonium chloride was dissolved in 12 mL dimethylsulfoxide. 3.41 g (85.3 mmol) sodium hydroxide was added and the mixture was heated to 60°C for one hour under a nitrogen atmosphere. 13.0 mL (13.64 g, 128 mmol) (2-chloroethyl)vinyl ether was added and the mixture was heated under a nitrogen atmosphere to 80°C for 6 hours. The reaction mixture was allowed to cool down to room temperature. 150 mL diethyl ether and 150 mL water were added. An oily layer was formed. The oily layer was isolated and washed with 50 mL diethyl ether. The oily residue was isolated and redissolved in 70 mL nitromethane. The mixture was extracted twice with 50 mL water, dried over Na₂SO₄ and evaporated under reduced pressure. The crude tris[4-(2-vinyloxyethoxy)phenyl]sulfonium chloride was purified on Kieselgel 60, using chloroform/methanol 3/2 as eluent. The isolated tris[4-(2-vinyloxyethoxy)phenyl]sulfonium chloride was dissolved in 16 mL methanol and a solution of 6.5 g sodium triflate in 30 mL methanol was added. The mixture was stirred for 30 minutes. The precipitated residues were removed by filtration and the solution was evaporated to half its volume. 90 mL water was added and the mixture was extracted twice with 60 mL ethyl acetate. The pooled organic fractions were dried over Na₂SO₄ and evaporated under reduced pressure. 4.69 g (59 %) of tris[4-(2-vinyloxyethoxy)phenyl]sulfonium triflate was isolated as an viscous oil.

### Example 2

This example illustrates the reduction of volatile, extractable residues, when using a sulfonium initiator according to the present invention.

### Preparation of the Pigment dispersion DISP-1

The concentrated pigment dispersion DISP-1 was prepared by mixing the 140.0 g of the pigment PB15:4 and 466.7 g of a 30% solution in VEEAof the polymeric dispersant SP35000 for 30 minutes using a DISPERLUX™ YELLOW075 (from DISPERLUX S.A.R.L., Luxembourg) and subsequently milling this mixture in a Eiger Lab Bead mill (from EIGER TORRANCE Ltd.) using yttrium-stabilized zirconium oxide-beads of 0.4 mm diameter ("high wear resistant zirconia grinding media" from TOSOH Co.). The bead mill is filled for 52% with the grinding beads and water-cooled during milling at 4250 rpm for 100 minutes. After milling the dispersion was separated from the beads using a filter cloth.

The resulting concentrated pigment dispersion DISP-11 according to Table 1 exhibited an average particle size of 106 nm and a viscosity of 139 mPa.s..

**Table 1**

| **Component** | **wt%** |
|---|---|
| **PB15:4** | 15 |
| **SP35000** | 15 |
| **Genorad^{™} 16** | 1 |
| **VEEA** | 69 |

### Preparation of Radiation Curable Compositions

The comparative radiation curable composition COMP-1 and inventive radiation curable composition INV-1 were prepared according to Table 2. The weight% (wt%) was based on the total weight of the radiation curable composition.

**Table 2**

| **wt% of component:** | **COMP-1** | **INV-1** |
|---|---|---|
| **VEEA** | 42.5 | 42.5 |
| **Uvacure^{™} 1503** | 38.9 | 38.9 |
| **ITX** | 1.0 | 1 |
| **COMPINI-1** | 5.0 | - |
| **Sulfonium-1** | - | 5.0 |
| **DISP-1** | 12.5 | 12.5 |
| **Silwet^{™} L7602** | 0.1 | 0.1 |

The radiation curable compositions COMP-1 and INV-1 were coated on an unsubbed 100µm PET100 substrate, using a bar coater and a 10 µm wired bar. The coatings were cured on a Fusion DRSE-120 conveyer, equipped with a Fusion VPS/I600 lamp (D-bulb), which transported the samples under the UV-lamp on a conveyer belt at a speed of 20 m/min. The samples were coated twice under ambient atmosphere at full power of the lamp and once under nitrogen inertization at full power of the lamp. Nitrogen inertization was effected according to the following procedure.

Before a coated sample was placed on the conveyer belt, the coated sample was mounted on a metal plate and on top of the plate a metal frame of 1 cm height with a non UV-absorbing quartz glass window was placed, so that a sealed chamber was formed with the coated sample inside. Then, the trapped air in the chamber was replaced by nitrogen gas by introducing pure nitrogen gas into the chamber for 30 seconds.

The structure and the amount of volatile extractables in both the comparative radiation curable composition COMP-1 and inventive radiation curable composition INV-1 after curing is determined on fully cured coatings by a direct thermal desorption method, i.e. without sample preparation. The fully cured coating on a PET100 substrate having a backing layer was analysed with a Gerstel™ TDS2 ThermoDesorption System from Gerstel Gmbh & Co. KG using as operation conditions: 0.5 cm² of the cured coating was analyzed during 10 minutes at 150°C with on-line GC evaluation of peak intensity for the desorbed components. The oven program was set to 40°C for 30 seconds, followed by a temperature increase at a rate of 15°C/minute until 320°C, and keeping the sample at 320°C for 5 minutes. The chromatographic column was a Db1 column from J&W (30m x 0.32mm, 1 mm film thickness); the carrier gas was He at a flow rate of 2 mL/min. The desorbed compounds were trapped on TenaxTA at -60°C.

In a first run, GCMS was used to identify the volatile degradation products of both the comparative and inventive initiator. The following volatile degradation products for the comparative initiator COMPINI-1 were detected for the comparative radiation curable composition COMP-1:

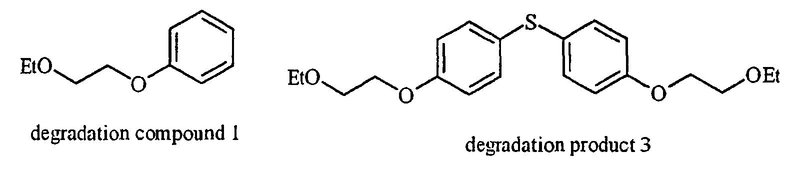

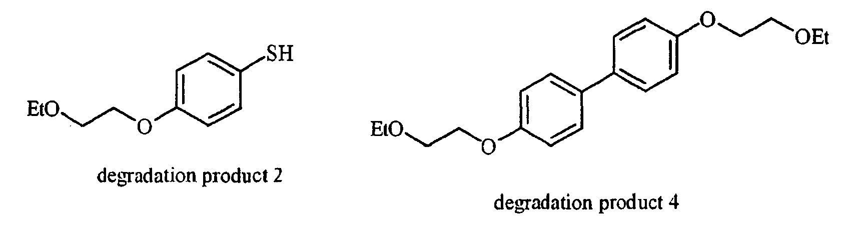

Using the same procedure, no degradation products of sulfonium-1 could be detected in the inventive radiation curable composition INV-1.

In a separate analysis the degradation compounds for COMP-1 were quantified according to the following procedure. The back coating on the PET100 substrate contained volatile compounds, including NMP. The amount of NMP per m² was determined independently. The amount of NMP detected was used as an internal standard to calculate the amount of volatile compounds from the cured coating expressed in ppm (mg extractable compound per kg). The amount of each degradation product was determined relative to the amount of NMP, using their relative peak area compared to NMP. The thermal desorption method and GC-conditions described above were used, in combination with a FID detector in stead of a mass spectrometer. An equal response to the GC-detector for each compound was assumed. The initial detector response in pAs was recalculated in ppm according to the following procedure. Based on an independent analysis of the NMP content, a response of 167pAs corresponds to 1 mg/m² NMP, which on its turn corresponds to 100 ppm for a 10 g/m² coating. The same ratio was also used to recalculate the amount of volatile degradation products to ppm. The results are summarized in Table 3.

**Table 3**

| **Degradation product** | **ppm** |
|---|---|
| Degradation product 1 | 115 |
| Degradation product 2 | 503 |
| Degradation product 3 | 1117 |
| Degradation product 4 | 1294 |

From this example, it should be clear that sulfonium initiators according to the present invention are superior in avoiding migratable volatile degradation products.

## Claims

1. A radiation curable composition comprising a cationic photoinitiator according to Formula (I) or (II): 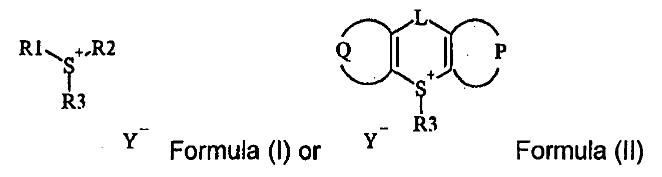 wherein :
R1, R2 and R3 independently represent a group selected from the group consisting of an aryl group and a heteroaryl group, with the proviso that R1, R2 and R3 are each substituted by at least one substituent comprising at least one cationically polymerizable group selected from the group consisting of an alkenyl ether, an epoxide and an oxetane;
P and Q independently represent the necessary atoms to form a substituted or unsubstituted 5 or 6-membered aromatic or heteroaromatic ring, with the proviso that at least one of P,Q and L is substituted by at least one substituent comprising at least one cationically polymerizable group, selected from the group consisting of an alkenyl ether, an epoxide and an oxetane;
L represents a group selected from the group consisting of a direct bond, O, S, SO, SO₂, CR5R6, C(=O), S(=O), C(NR8) and NR7;
R5 and R6 independently represent a group selected from the group consisting of a hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group, a substituted or unsubstituted aryl group, a substituted or unsubstituted heteroaryl group, a halogen, an alcohol, an ether, an ester, an amine, an amide, a carboxylic acid, a thiol and thioether;
R5 and R6 may represent the necessary atoms to form a five or six membered ring;
R7 and R8 independently represent a group selected from the group consisting of a hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group, a substituted or unsubstituted aryl group, a substituted or unsubstituted heteroaryl group and an acyl group; and
Y- represents a counter ion to compensate for the positive charge of the sulfonium compound,
wherein the composition contains one or more monomers, oligomers and prepolymers selected from the group consisting of epoxides, oxetanes and vinyl ethers; and
wherein the radiation curable composition does not contain an organic solvent or is substantially free of organic solvent.

2. The radiation curable composition according to claim 1 wherein R1, R2 and R3 all represent a phenyl group each substituted by at least one substituent comprising at least one cationically polymerizable group selected from the group consisting of an alkenyl ether, an epoxide and an oxetane.

3. The radiation curable composition according to claim 1 wherein the 6-membered aromatic rings formed through the atoms P and Q both represent a phenyl group;

4. The radiation curable composition according to any one of claims 1 to 3
wherein the at least one cationically polymerizable group is a vinyl ether group.

5. The radiation curable composition according to any one of claims 1 to 4
wherein the composition contains a colorant.

6. The radiation curable composition according to claim 5 wherein the colorant is a pigment.

7. The radiation curable composition according to any one of claims 1 to 6
wherein the monomers, oligomers and prepolymers are present in the range of 60 to 95 % by weight based on the total weight of radiation curable composition.

8. The radiation curable composition according to any one of claims 1 to 7
wherein the composition is a radiation curable inkjet composition.

9. The radiation curable composition according to any one of claims 1 to 7
wherein the composition is a radiation curable ink for offset printing, screen printing or flexographic printing.

10. A radiation curable ink set comprising radiation curable compositions as defined by any one of claims 1 to 9.

11. A method of making a printed article comprising the steps of:
a) providing a radiation curable composition as defined by any one of claims 1 to 8;
b) applying the radiation curable composition on a food packaging material.

12. The method according to claim 11 wherein the radiation curable composition is applied by inkjet printing.

13. Use of a radiation curable composition as defined by any one of claims 1 to 8 to reduce the amount of extractable degradation products of the photoinitiator after curing of the composition.

## Patentansprüche

1. Eine strahlungshärtbare Zusammensetzung, die einen kationischen Fotoinitiator gemäß Formel (I) oder Formel (II) enthält: 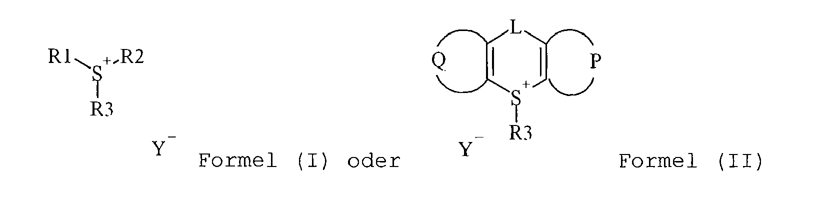 in der :
R1, R2 und R3 unabhängig voneinander eine Gruppe aus der Gruppe bestehend aus einer Arylgruppe und einer Heteroarylgruppe bedeuten, vorausgesetzt, dass R1, R2 und R3 jeweils durch mindestens einen Substituenten, der mindestens eine kationisch polymerisierbare Gruppe aus der Gruppe bestehend aus einem Alkenylether, einem Epoxid und einem Oxetan umfasst, substituiert sind,
P und Q unabhängig voneinander die zur Bildung eines gegebenenfalls substituierten 5- oder 6-gliedrigen aromatischen oder heteroaromatischen Ringes benötigten Atome bedeuten, vorausgesetzt, dass mindestens eines von P, Q und L durch mindestens einen Substituenten, der mindestens eine kationisch polymerisierbare Gruppe aus der Gruppe bestehend aus einem Alkenylether, einem Epoxid und einem Oxetan umfasst, substituiert ist,
L eine Gruppe aus der Gruppe bestehend aus einer direkten Binding, O, S, SO, SO₂, CR5R6, C(=O), S(=O), C(NR8) und NR7 ist,
R5 und R6 unabhängig voneinander eine Gruppe aus der Gruppe bestehend aus einem Wasserstoffatom, einer gegebenenfalls substituierten Alkylgruppe, einer gegebenenfalls substituierten Alkenylgruppe, einer gegebenenfalls substituierten Alkynylgruppe,
einer gegebenenfalls substituierten Aralkylgruppe, einer gegebenenfalls substituierten Alkarylgruppe, einer gegebenenfalls substituierten Arylgruppe, einer gegebenenfalls substituierten Heteroarylgruppe, einem Halogen, einem Alkohol, einem Ether, einem Ester, einem Amin, einem Amid, einer Carbonsäure, einem Thiol und einem Thioether bedeuten,
R5 und R6 die zur Bildung eines 5- oder 6-gliedrigen Ringes benötigten Atome bedeuten können,
R7 und R8 unabhängig voneinander eine Gruppe aus der Gruppe bestehend aus einem Wasserstoffatom, einer gegebenenfalls substituierten Alkylgruppe, einer gegebenenfalls substituierten Alkenylgruppe, einer gegebenenfalls substituierten Alkynylgruppe, einer gegebenenfalls substituierten Aralkylgruppe, einer gegebenenfalls substituierten Alkarylgruppe, einer gegebenenfalls substituierten Arylgruppe, einer gegebenenfalls substituierten Heteroarylgruppe und einer Acylgruppe bedeuten, und
Y⁻ ein Gegenion zur Ausgleichung der positiven Ladung der Sulfoniumverbindung bedeutet,
wobei die Zusammensetzung ein oder mehrere Monomere, Oligomere und Prepolymere aus der Gruppe bestehend aus Epoxiden, Oxetanen und Vinylethern enthält, und
wobei die strahlungshärtbare Zusammensetzung kein organisches Lösungsmittel enthält oder im Wesentlichen kein organisches Lösungsmittel enthält.

2. Strahlungshärtbare Zusammensetzung nach Anspruch 1, **dadurch gekennzeichnet, dass** R1, R2 und R3 alle eine Phenylgruppe bedeuten, die jeweils durch mindestens einen Substituenten, der mindestens eine kationisch polymerisierbare Gruppe aus der Gruppe bestehend aus einem Alkenylether, einem Epoxid und einem Oxetan umfasst, substituiert ist.

3. Strahlungshärtbare Zusammensetzung nach Anspruch 1, **dadurch gekennzeichnet, dass** die durch die Atome P und Q gebildeten 6-gliedrigen aromatischen Ringe beide eine Phenylgruppe bedeuten.

4. Strahlungshärtbare Zusammensetzung nach einem der Ansprüche 1 bis 3, **dadurch gekennzeichnet, dass** die mindestens eine kationisch polymerisierbare Gruppe eine Vinylethergruppe ist.

5. Strahlungshärtbare Zusammensetzung nach einem der Ansprüche 1 bis 4, **dadurch gekennzeichnet, dass** die Zusammensetzung ein Farbmittel enthält.

6. Strahlungshärtbare Zusammensetzung nach Anspruch 5, **dadurch gekennzeichnet, dass** das Farbmittel ein Pigment ist.

7. Strahlungshärtbare Zusammensetzung nach einem der Ansprüche 1 bis 6, **dadurch gekennzeichnet, dass** die Monomere, Oligomere und Prepolymere in einem Verhältnis von 60 bis 95 Gew.-%, bezogen auf das Gesamtgewicht der strahlungshärtbaren Zusammensetzung, enthalten sind.

8. Strahlungshärtbare Zusammensetzung nach einem der Ansprüche 1 bis 7, **dadurch gekennzeichnet, dass** die Zusammensetzung eine strahlungshärtbare Tintenstrahlzusammensetzung ist.

9. Strahlungshärtbare Zusammensetzung nach einem der Ansprüche 1 bis 7, **dadurch gekennzeichnet, dass** die Zusammensetzung eine strahlungshärtbare Tinte für Offsetdruck, Siebdruck oder flexografischen Druck ist.

10. Ein Satz strahlungshärtbarer Tinten, die wie nach einem der Ansprüche 1 bis 9 definierte strahlungshärtbare Zusammensetzungen umfasst.

11. Ein Verfahren zur Herstellung eines gedruckten Artikels, das folgende Schritte umfasst :
a) Schaffen einer wie nach einem der Ansprüche 1 bis 8 definierten strahlungshärtbaren Zusammensetzung,
b) Auftrag der strahlungshärtbaren Zusammensetzung auf ein Nahrungsverpackungsmaterial.

12. Verfahren nach Anspruch 11, **dadurch gekennzeichnet, dass** die strahlungshärtbare Zusammensetzung durch Tintenstrahldruck aufgetragen wird.

13. Verwendung einer wie nach einem der Ansprüche 1 bis 8 definierten strahlungshärtbaren Zusammensetzung, um die Menge extrahierbarer Zersetzungsprodukte des Fotoinitiators nach Härtung der Zusammensetzung zu beschränken.

## Revendications

1. Composition durcissable par rayonnement contenant un photoinitiateur cationique répondant à la Formule (I) ou (II) : 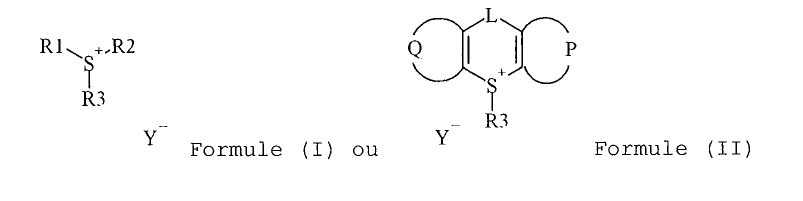 où :
R1, R2 et R3 représentent, indépendamment l'un de l'autre, un groupe choisi parmi le groupe composé d'un groupe aryle et d'un groupe hétéroaryle, à condition que R1, R2 et R3 soient chacun substitués par au moins un substituant comprenant au moins un groupe cationiquement polymérisable choisi parmi le groupe composé d'un éther d'alkényle, d'un époxyde et d'un oxétane,
P et Q représentent, indépendamment l'un de l'autre, les atomes nécessaires pour former un noyau aromatique ou hétéroaromatique pentagonal ou hexagonal éventuellement substitué, à condition qu'au moins l'un de P, Q et L soit substitué par au moins un substituant comprenant au moins un groupe cationiquement polymérisable choisi parmi le groupe composé d'un éther d'alkényle, d'un époxyde et d'un oxétane,
L représente un groupe choisi parmi le groupe composé d'une liaison directe, O, S, SO, SO₂, CR5R6, C(=O), S(=O), C(NR8) et NR7,
R5 et R6 représentent, indépendamment l'un de l'autre, un groupe choisi parmi le groupe composé d'un atome d'hydrogène, d'un groupe alkyle éventuellement substitué, d'un groupe alkényle éventuellement substitué, d'un groupe alkynyle éventuellement substitué, d'un groupe aralkyle éventuellement substitué, d'un groupe alkaryle éventuellement substitué, d'un groupe aryle éventuellement substitué, d'un groupe hétéroaryle éventuellement substitué, d'un halogène, d'un alcool, d'un éther, d'un ester, d'une amine, d'un amide, d'un acide carboxylique, d'un thiol et d'un thioéther,
R5 et R6 peuvent représenter les atomes nécessaires pour former un noyau pentagonal ou hexagonal,
R7 et R8 représentent, indépendamment l'un de l'autre, un groupe choisi parmi le groupe composé d'un atome d'hydrogène, d'un groupe alkyle éventuellement substitué, d'un groupe alkényle éventuellement substitué, d'un groupe alkynyle éventuellement substitué, d'un groupe aralkyle éventuellement substitué, d'un groupe alkaryle éventuellement substitué, d'un groupe aryle éventuellement substitué, d'un groupe hétéroaryle éventuellement substitué et d'un groupe acyle, et
Y⁻ représente un contre-ion compensant la charge positive du composé de sulfonium,
ladite composition contenant un ou plusieurs monomères, oligomères et prépolymères choisis parmi le groupe composé d'époxydes, d'oxétanes et d'éthers vinyliques, et
ladite composition durcissable par rayonnement ne contenant pas de solvant organique ou ne contenant essentiellement pas de solvant organique.

2. Composition durcissable par rayonnement selon la revendication 1, **caractérisée en ce que** R1, R2 et R3 représentent chacun un groupe phényle substitué par au moins un substituant comprenant au moins un groupe cationiquement polymérisable choisi parmi le groupe composé d'un éther d'alkényle, d'un époxyde et d'un oxétane.

3. Composition durcissable par rayonnement selon la revendication 1, **caractérisée en ce que** les noyaux aromatiques hexagonaux formés par les atomes P et Q représentent chacun un groupe phényle.

4. Composition durcissable par rayonnement selon l'une quelconque des revendications 1 à 3, **caractérisée en ce que** ledit au moins un groupe cationiquement polymérisable est un groupe éther vinylique.

5. Composition durcissable par rayonnement selon l'une quelconque des revendications 1 à 4, **caractérisée en ce que** la composition contient une matière colorante.

6. Composition durcissable par rayonnement selon la revendication 5, **caractérisée en ce que** la matière colorante est un pigment.

7. Composition durcissable par rayonnement selon l'une quelconque des revendications 1 à 6, **caractérisée en ce que** les monomères, oligomères et prépolymères sont contenus dans un rapport de 60 à 95% en poids par rapport au poids total de la composition durcissable par rayonnement.

8. Composition durcissable par rayonnement selon l'une quelconque des revendications 1 à 7, **caractérisée en ce que** la composition est une composition à jet d'encre durcissable par rayonnement.

9. Composition durcissable par rayonnement selon l'une quelconque des revendications 1 à 7, **caractérisée en ce que** la composition est une encre durcissable par rayonnement pour impression offset, sérigraphie ou impression flexographique.

10. Un ensemble d'encres durcissables par rayonnement comprenant des compositions durcissables par rayonnement telles que définies selon l'une quelconque des revendications 1 à 9.

11. Un procédé pour la confection d'un article imprimé, comprenant les étapes consistant à :
a) mettre à disposition une composition durcissable par rayonnement telle que définie selon l'une quelconque des revendications 1 à 8,
b) appliquer la composition durcissable par rayonnement sur un matériau d'emballage d'aliments.

12. Procédé selon la revendication 11, **caractérisé en ce que** la composition durcissable par rayonnement est appliquée par impression à jet d'encre.

13. L'utilisation d'une composition durcissable par rayonnement telle que définie selon l'une quelconque des revendications 1 à 8, afin de réduire la quantité de produits de décomposition extractibles du photoinitiateur qui sont présents après le durcissement de la composition.
